Europäisches Patentamt

European Patent Office

Office européen des brevets

(19)

(11) Numéro de publication: **0 558 509 B1**

(12) **FASCICULE DE BREVET EUROPEEN**

(45) Date de publication de fascicule du brevet: **15.03.95**    (51) Int. Cl.6: **A61K 7/48**, A61K 7/06, A61K 35/78, //(A61K35/78, 31:07,31:68,31:355,31:49, 31:505,31:56)

(21) Numéro de dépôt: **91918406.9**

(22) Date de dépôt: **18.10.91**

(86) Numéro de dépôt internationale : **PCT/FR91/00818**

(87) Numéro de publication internationale : **WO 92/09262 (11.06.92 92/13)**

(54) UTILISATION DE SAPONINES DE MEDICAGO POUR LA PREPARATION DE COMPOSITIONS COSMETIOUES OU PHARMACEUTIOUES, NOTAMMENT DERMATOLOGIOUES FAVORISANT LE RENOUVELLEMENT DE L'EPIDERME, STIMULANT LA REPOUSSE DES CHEVEUX, OU RETARDANT LEUR CHUTE.

(30) Priorité: **21.11.90 FR 9014542**

(43) Date de publication de la demande: **08.09.93 Bulletin 93/36**

(45) Mention de la délivrance du brevet: **15.03.95 Bulletin 95/11**

(84) Etats contractants désignés: **BE CH DE ES FR GB IT LI**

(56) Documents cités: **FR-A- 2 187 328**

**DICTIONNAIRE VIDAL, Editions Vidal, Paris (FR), 1992, p. 912: "Minoxidil Gerbiol"**

**Chemical Abstracts, vol. 90, no. 12, 19 March 1979, (Colombus, Ohio, US), page 304, abstract 92411u, & US-A-865985 (Malinow, M.R.) 7 July 1978**

(73) Titulaire: **LVMH RECHERCHE 48-50, rue de Seine, B.P. 79 F-92703 Colombes Cédex (FR)**

(72) Inventeur: **BONTE, Frédéric 5, place Charras F-92400 Courbevoie (FR)** Inventeur: **MEYBECK, Alain Les Poissons 20 ter, rue de Bezons F-92400 Courbevoie (FR)** Inventeur: **MASSIOT, Georges 1, impasse du Levant F-51100 Reims (FR)**

(74) Mandataire: **Portal, Gérard et al Cabinet Beau de Loménie 158, rue de l'Université F-75340 Paris Cédex 07 (FR)**

EP 0 558 509 B1

Chemical Abstracts, vol. 75, no. 8, 23 August 1971 (Colombus, Ohio, US) B. Gestetner et al.: "Lucerne saponina. IV. Relation between their chemical constitution and hemolytic and antifungal activities", page 237, abstract 52715e, & J. Sci. Food, Agr. 1971, 22(4), 168-72

## Description

La présente invention concerne essentiellement l'utilisation de saponines de Medicago ou des sapogénines correspondantes pour la préparation de compositions cosmétiques ou pharmaceutiques, notamment dermatologiques, destinées en particulier à favoriser le renouvellement de l'épiderme, à stimuler la repousse des cheveux ou à retarder leur chute, et à lutter contre les effets du vieillissement sur l'état de la peau et de la chevelure.

Les plantes du genre Medicago, désignées souvent par le terme générique "luzernes", sont des Légumineuses largement répandues sur la planète dans les zones tempérées et dans certaines régions arides, soit à l'état sauvage soit à l'état cultivé comme fourrage pour les animaux.

Medicago sativa ou alfalfa, la luzerne proprement dite, est le principal représentant de cette famille. Par des plantes du genre Medicago sont présentes sur les cinq continents notamment en France, dans le bassin méditerranéen, aux Etats-Unis, au Canada et en Australie. Parmi les autres espèces de Medicagos, citons : M. lupulina (Canada), M. truncatula (Australie, Afrique du Sud), M. laciniata (zones arides et semiarides d'Australie, Arabie Saoudite, (Lybie), M. littoralis (Australie), M. minima (Algérie), M. falcata (URSS, Canada), M. media (Alaska), M. Arborea (Grèce).

Les luzernes contiennent une grande variété de substances utiles à l'alimentation animale et humaine, notamment des protéines, des vitamines, des caroténoïdes, des sels minéraux (J.G. COORS et al., Crop Science, 1986, vol. 26, n° 5, p. 843-848 ; E.M. BICKOFF et al. in Alfalfa Science an Technology, ed. C.H. HANSON, publiée par The American Society of Agronomy, Madison, Wisconsin USA, 1972, p. 247-282). Les feuilles et surtout les racines contiennent également des composés glycosylés constitués essentiellement de saponines triterpéniques décrites en particulier par G. MASSIOT et al., J. Chem. Soc. Perkin Trans. I (1988) p. 3071-3079. L'hydrolyse des liaisons glycosidiques de ces saponines conduit à l'obtention des sapogénines triterpéniques correspondantes, dont la plus abondante est l'acide médicagénique (G. Massiot et al., J. Agric. Food Chem., 1988, vol. 36, p. 902-909). Enfin, des glycosides stéroliques, présents en très faible quantité, ont été identifiés par S. ITO et al., Nippon Nogei Kagaku Kaishi 1973, vol. 47, n° 3, p. 229-230, en particulier le bêta-sitostéryl glucoside et le stigmastéryl glucoside.

Un certain nombre d'utilisations thérapeutiques d'extraits ou de substances extraites de la luzerne - Medicago sativa - ont été décrites.

Ainsi on a recommandé l'administration du suc de luzerne pour traiter les avitaminoses, la décalcification (Aldo Poletti "Fleurs et Plantes médicinales" ed. Delachaux et Niestlé p. 126), l'extrait de graines a été décrit comme possédant une activité anti-inflammatoire dans le document SU-624 634. Egalement, un extrait de luzerne a été décrit dans le document FR-2 571 256 comme possédant une activité oestrogénique, trouvant son application dans le traitement de la cellulite. L'acide médicagénique précité possède une activité hémolytique (B. GESTETNER, et col., Experientia, 1971, 27 (1) 40-41) et antifongique (DE-3 717 280).

Enfin l'utilisation en cosmétique des glucosides stéroliques présents dans les feuilles a été décrite dans le document JP-62-72 604 pour favoriser l'hydratation de la peau.

Les effets précités du vieillissement sur la peau se caractérisent en particulier par un ralentissement de la différenciation cellulaire de l'épiderme, notamment des kératinocytes, d'où un ralentissement de leur renouvellement et de leur activité, ce qui entraîne un aspect de la peau plus terne, desséché et plus ridé. Les effets du vieillissement au niveau des follicules pileux sont également négatifs. Ils entraînent pour les kératinocytes des follicules, comme pour ceux de l'épiderme, une diminution de leur activité, d'où un ralentissement de la croissance des cheveux, et, à terme, une dégénérescence du follicule et la perte définitive du cheveu.

La présente invention a pour but de résoudre le nouveau problème technique consistant en la fourniture d'une nouvelle formulation d'une composition cosmétique ou pharmaceutique, notamment dermatologique, présentant une bonne efficacité sur le renouvellement de l'épiderme, la repousse des cheveux, la prévention ou le ralentissement de leur chute, ainsi que dans la lutte contre les effets du vieillissement sur l'état de la peau et de la chevelure.

La présente invention a encore pour but de résoudre ce nouveau problème technique d'une manière particulièrement simple, utilisable à l'échelle industrielle.

La présente invention permet de résoudre ce problème technique pour la première fois, d'une manière satisfaisante, utilisable à l'échelle industrielle.

Ainsi, selon un premier aspect, la présente invention concerne l'utilisation d'au moins une saponine triterpénique de Medicago ou d'au moins une sapogénine correspondante ou d'un extrait végétal en contenant, pour la fabrication d'une composition cosmétique ou pharmaceutique, notamment dermatologique, destinée en particulier à favoriser le renouvellement de l'épiderme, à stimuler la repousse des cheveux ou à retarder leur chute et à lutter contre les effets du vieillissement sur l'état de la peau et de la chevelure.

Selon une variante de réalisation particulière, la saponine et l'extrait végétal précités sont obtenus par extraction à partir de parties aériennes, telles que feuilles ou tiges, ou de racines de Medicago, de préférence à partir de racines de cette plante. De préférence encore, les parties de la plante utilisées sont séchées préalablement au traitement d'extraction.

Suivant une autre variante, la saponine et l'extrait végétal précités sont obtenus par extraction à partir de cals obtenus par culture in vitro de tissus de Medicago, en particulier à partir de tissus de racines de cette plante, par exemple suivant la technique décrite par BESSON V. et al. dans Phytochemistry 1989 vol. 28, n° 5, pages 1379 et 1380.

Suivant une variante avantageuse, la saponine précitée est choisie parmi celles qui comportent un groupe carboxylique, et est utilisée sous forme acide pour la mise en oeuvre de la présente invention.

Selon un autre mode de réalisation, la sapogénine précitée est de préférence choisie parmi le groupe constitué par l'acide lucernique, l'acide médicagénique, l'acide zanhique, la bayogénine, l'hédéragénine, les sojasapogénols A, B, C et E.

Suivant encore une autre variante de réalisation de l'invention, la plante Medicago précitée est choisie parmi le groupe constitué par : Medicago sativa, Medicago lupulina, Medicago truncatula, Medicago laciniata, Medicago littoralis, Medicago falcata, Medicago media, Medicago minima, Medicago varia, Medicago arborea et Medicago romanica.

Selon encore un mode particulier de réalisation de l'invention, l'extrait végétal précité est obtenu selon le procédé décrit ci-après à titre indicatif, mais nullement limitatif. On extrait la matière sèche, constituée de préférence de racines de Medicago, au moyen d'un solvant choisi parmi le groupe constitué par : l'eau, les alcools comportant de préférence de 1 à 4 atomes de carbone, les esters organiques comportant de préférence de 3 à 6 atomes de carbone ; ou d'un solvant mixte à base d'un mélange quelconque des solvants précités.

Avantageusement, le solvant d'extraction primaire est le méthanol, l'éthanol, un mélange méthanol-eau ou un mélange éthanol-eau.

Le rapport de la matière végétale à l'agent d'extraction n'est pas critique et généralement sera compris entre 1 : 5 et 1 : 20 parties en poids.

L'extraction primaire précitée s'effectue à des températures comprises entre la température ambiante et le point d'ébullition du solvant utilisé pour l'extraction.

De préférence, l'extraction primaire est effectuée au reflux sous pression atmosphérique pendant une durée de 2 à 4 h. En outre, elle est avantageusement précédée d'une macération à froid pendant 2 à 4 h dans le solvant d'extraction.

En fin d'extraction, la phase du solvant contenant l'extrait est filtrée puis concentrée et/ou évaporée à sec sous pression réduite. On obtient ainsi un premier extrait selon l'invention riche en saponines.

Suivant une variante particulière, l'utilisation selon l'invention est relative à un mélange de saponines précitées. On obtient en particulier un mélange de saponines selon l'invention à partir du premier extrait concentré ou sec précité en opérant comme indiqué ci-après. Le premier extrait précité est introduit puis agité dans un solvant apolaire de préférence miscible avec le solvant d'extraction primaire, tel qu'un éther ou une cétone de faible poids moléculaire, en particulier l'éther éthylique ou isopropylique, l'acétone ou la méthyl-éthyl-cétone. La quantité de solvant apolaire est, en poids, généralement de 5 à 100 parties pour une partie d'extrait primaire. L'insoluble et/ou le précipité formé contient principalement un mélange de saponines selon l'invention.

Avantageusement, le mélange de saponines obtenu précédemment est purifié par un procédé quelconque à la portée de l'homme de l'art.

En particulier l'insoluble et/ou le précipité précité est remis en solution dans 20 fois environ son poids d'eau. La solution aqueuse est ensuite extraite 3 à 4 fois par un alcool peu soluble dans l'eau, tel que le butanol saturé en eau, par exemple en proportion 1/1 en volume à chaque opération d'extraction. Les phases alcooliques sont réunies et évaporées sous pression réduite. Le résidu est dissous dans 10 fois environ son poids d'eau, la solution est ensuite dialysée contre de l'eau pure pendant 4 à 5 jours. Le contenu de la cellule de dialyse est lyophilisé. Eventuellement pour améliorer encore la purification du mélange de saponines obtenu, le lyophilisat est dissous dans du méthanol, puis jeté dans de l'éther éthylique. On recueille le précipité forme.

Avantageusement, le mélange de saponines obtenu subit un traitement supplémentaire, consistant par exemple en un passage dudit mélange en solution aqueuse sur une résine acide échangeuse de cations, suivi d'une élution par de l'eau ou par un mélange méthanol-eau, ledit traitement supplémentaire étant destiné à faire passer sous forme acide les saponines qui comportent un groupement carboxylique salifié.

Les sapogénines selon l'invention précitées sont obtenues de préférence à partir des saponines extraites selon le procédé précédemment décrit. A cet effet on réalise l'hydrolyse des liaisons glycosidiques desdites saponines. Avantageusement, on réalise une hydrolyse acide, notamment avec des acides halogénés tels que l'acide perchlorique, l'acide fluoborique, l'acide trifluoroacétique, comme décrit par exemple dans la publication de G. Massiot et al. dans Journal of Agricultural and food Chemistry 1988, vol. 36, p. 902-909.

Selon une autre variante de réalisation avantageuse de l'invention, la saponine précitée ou la sapogénine correspondante précitée, ou l'extrait de luzerne précité est au moins en partie incorporé dans une phase lamellaire lipidique hydratée ou dans des vésicules de type liposomes.

Le terme "lipidique" dans l'expression "phase lamellaire lipidique" couvre toutes les substances comprenant une chaîne carbonée dite grasse, comportant généralement plus de 5 atomes de carbone, cette substance étant habituellement dénommée "lipide".

Selon l'invention, on utilise à titre de lipides, pour former la phase lamellaire lipidique, ou les vésicules type liposomes, des lipides amphiphiles, c'est-à-dire constitués de molécules possédant un groupe hydrophile indifféremment ionique ou non ionique et un groupe lipophile, ces lipides amphiphiles étant susceptibles de former une phase lamellaire lipidique ou des vésicules type liposomes, en présence d'une phase aqueuse, selon la quantité d'eau dans le mélange.

En particulier, parmi ces lipides, on peut citer : les phospholipides, les phosphoaminolipides, les glycolipides, les alcools gras polyoxyéthylénés, les esters de polyol éventuellement polyoxyéthylénés. De telles substances sont par exemple constituées par une lécithine d'oeuf ou de soja, une phosphatidylsérine, une sphyngomyéline, un cérébroside, un glycosyl céramide ou un stéarate de polyglycérol oxyéthyléné.

De préférence, selon l'invention, on utilise un mélange lipidique constitué d'au moins un lipide amphiphile et d'au moins un lipide hydrophobe tel que stérol, comme le cholestérol ou le bêta-sitostérol. La quantité, exprimée en poids, de composés hydrophobes ne doit généralement pas être supérieure à la quantité de lipides amphiphiles, et de préférence ne doit pas être supérieure à 0,5 fois cette quantité.

Suivant une variante préférée, la saponine précitée est introduite dans la phase aqueuse de la phase lamellaire lipidique hydratée ou des liposomes à une concentration comprise entre 0,01% et 5% en poids du poids total de ladite phase aqueuse.

Suivant encore une autre variante préférée, la sapogénine précitée est incorporée dans la phase lipidique de la phase lamellaire lipidique hydratée ou des liposomes à une concentration comprise de préférence entre 0,01% et 30% en poids de ladite phase lipidique. Dans ce cas il n'est généralement pas nécessaire d'ajouter dans la phase lipidique un autre constituant hydrophobe, tel qu'un stérol. La concentration précitée est de préférence encore comprise entre 0,01% et 10% en poids de cette phase lipidique.

L'incorporation dans des phases lamellaires lipiques hydratées ou dans des liposomes, des saponines ou des sapogénines précitées ou des extraits précités selon l'invention, peut être réalisée selon les techniques de préparation connues, décrites par exemple dans le document EP-B1-0087 993 = US-A-4 508 703 ; et éventuellement en combinaison avec le document EP-B1-00101 559 = US-A-4 621 023.

Selon un second aspect, la présente invention concerne aussi une composition cosmétique ou pharmaceutique, notamment dermatologique, destinée en particulier à favoriser le renouvellement de l'épiderme, à stimuler la repousse des cheveux ou à retarder leur chute et à lutter contre les effets du vieillissement sur l'état de la peau et de la chevelure, caractérisée en ce qu'elle comprend, à titre d'ingrédient actif, une quantité efficace d'au moins une saponine triterpénique de Medicago ou d'au moins une sapogénine correspondante ou d'au moins un extrait végétal en contenant, éventuellement dans un excipient support ou véhicule cosmétiquement ou pharmaceutiquement acceptable.

De préférence, la saponine précitée, ou la sapogénine précitée, ou l'extrait précité est obtenu à partir de parties aériennes telles que feuilles ou tiges, ou de racines de Medicago, de préférence à partir de racines de cette plante.

Diverses variantes de réalisation de la composition découlent clairement de la description précédente relativement à l'utilisation.

En particulier, la saponine ou la sapogénine précitées ou l'extrait végétal précité peuvent avantageusement être au moins en partie incorporés dans des phases lamellaires lipidiques hydratées ou dans des vésicules de type liposomes.

Par ailleurs, selon une variante de réalisation avantageuse, la concentration en saponine ou sapogénine précitées ou en extrait végétal précité est de préférence comprise entre 0,001% et 5%, encore mieux entre 0,01 et 2% en poids, par rapport au poids total de la composition cosmétique ou pharmaceutique.

Ces proportions s'entendent en poids sec lorsqu'il s'agit des extraits végétaux.

Selon une autre variante avantageuse de l'invention, la composition cosmétique ou pharmaceutique, notamment dermatologique selon l'invention, comprend en outre au moins une autre substance active, en une concentration efficace, choisie parmi les xanthines, les vitamines, en particulier les vitamines A, B et E, la tyrosine ou ses dérivés comme par exemple le tyrosinate de glucose, la malyl tyrosine, la quinine ou ses dérivés, les rubéfiants tels que le nicotinate de méthyle, un surnageant de culture de fibroblaste de papilles, tel que décrit dans le document EP-A-272 920, des hydrolysats de kératine, des oligo-éléments tels que zinc, sélénium, cuivre, des inhibiteurs de 5-$\alpha$-réductase tels que : progestérone, acétate de cyprotérone, Minoxidil®, acide azélaïque et ses dérivés, 1,4-méthyl-4-azastéroïde, en particulier la 17-$\beta$-N,N-diéthylcarbamoyl-4-méthyl4-aza-5-$\alpha$-androstan-3-one, ou encore un extrait de Serenoa repens. Avantageusement, cette substance active peut être incorporée au moins en partie dans des phases lamellaires lipidiques hydratées ou dans des vésicules type liposomes.

Les compositions cosmétiques ou pharmaceutiques, notamment dermatologiques, conformément à la présente invention peuvent être appliquées par voie topique en particulier pour favoriser le renouvellement de l'épiderme, stimuler la repousse des cheveux, ou retarder leur chute, et pour lutter contre les effets du vieillissement sur l'état de la peau et de la chevelure, en particulier dans des compositions se présentant sous forme de crèmes, de gels ou de lotions destinés à l'application topique.

Sous cet aspect, la présente invention fournit également un procédé de traitement cosmétique destiné notamment à favoriser le renouvellement de l'épiderme, à stimuler la repousse des cheveux ou à retarder leur chute et à lutter contre les effets de vieillissement sur l'état de la peau et de la chevelure, caractérisé en ce qu'il comprend l'application topique, en une quantité efficace, pour réaliser ledit effet recherché, d'au moins une saponine de Medicago ou d'au moins une sapogénine correspondante ou d'un extrait végétal en contenant. Selon une variante de réalisation avantageuse, ladite saponine ou sapogénine précitées ou ledit extrait végétal est au moins en partie incorporé dans une phase lamellaire lipidique hydratée ou dans des vésicules de type liposomes.

Il est à noter que dans l'expression "au moins en partie dans des phases lamellaires lipidiques hydratées ou dans des vésicules type liposomes" on entend dans la présente description et dans les revendications que l'ingrédient actif précité est combiné à des phases lamellaires lipidiques hydratées ou à des vésicules type liposomes, quelle que soit la forme de cette combinaison.

Cependant, il est clair qu'une telle combinaison peut résider dans l'incorporation, ou même l'encapsulation dans les phases lamellaires lipidiques hydratées ou dans les vésicules type liposomes. Mais il n'est pas nécessaire que la totalité de cet ingrédient actif soit incorporée ou encapsulée pour obtenir l'effet recherché notamment sur l'épiderme et sur la chevelure.

Selon un autre aspect, l'invention fournit encore un procédé de fabrication d'une composition cosmétique ou pharmaceutique, notamment dermatologique, destinée en particulier à favoriser le renouvellement de l'épiderme, à stimuler la repousse des cheveux ou à retarder leur chute et à lutter contre les effets du vieillissement sur l'état de la peau et de la chevelure, caractérisé en ce qu'il comprend l'emploi d'au moins une saponine de Medicago ou d'au moins une sapogénine correspondante ou d'un extrait végétal en contenant, que l'on mélange dans un excipient, véhicule ou support pharmaceutiquement ou cosmétiquement acceptable. Selon une variante de réalisation, ce procédé comprend en premier lieu l'incorporation d'au moins une saponine précitée ou d'au moins une sapogénine correspondante ou d'un extrait végétal en contenant, au moins en partie dans des phases lamellaires lipidiques hydratées ou dans des vésicules type liposomes, puis leur mélange dans un excipient, véhicule ou support pharmaceutiquement ou cosmétiquement acceptable.

D'autres buts, caractéristiques et avantages de l'invention apparaîtront clairement à la lecture de la description explicative qui va suivre, faite en référence à plusieurs exemples donnés uniquement à titre d'illustration et qui ne sauraient par conséquent d'aucune façon limiter la portée de l'invention.

Dans les exemples, les pourcentages sont exprimés en poids, sauf indication contraire. Dans le cas des extraits, ceux-ci sont exprimés en poids sec de l'extrait.

Exemple 1

Préparation d'un extrait de racines de luzerne (Medicago sativa) riche en saponines

200 g de racines de luzerne sèches et pulvérisées sont mis à macérer pendant 2 h dans 1,2 l de méthanol. Le mélange est porté au reflux pendant 3 h. Après refroidissement, la solution est filtrée sur verre fritté n° 3. Les marcs sont extraits de nouveau de la même façon par 2 fois 1 l de méthanol. Les trois filtrats sont réunis et concentrés à l'évaporateur rotatif jusqu'à un volume voisin de 500 ml.

On obtient ainsi un premier extrait brut riche en saponines, que l'on purifie de la manière suivante.

La solution concentrée est additionnée rapidement à 2,5 l d'acétone distillée ; le précipité qui se forme est recueilli sur verre fritté n° 4, lavé à l'acétone et essoré. Le précipité est mis dans un dessiccateur

(CaCl$_2$) et est maintenu sous pression réduite au moyen d'une pompe à palettes pendant plusieurs heures. Rendement = 18 g (9%).

Selon une variante, on peut remplacer le méthanol dans le procédé décrit ci-dessus par un mélange méthanol-eau à 20%. Toutefois, le rendement est dans ce cas légèrement inférieur.

Exemple 2

Préparation d'un mélange de saponines de racines de luzerne (Medicago sativa)

On prend 1 kg de poudre de racines de luzerne que l'on met à macérer dans 6 l de méthanol pendant 2 h. La suspension est portée au reflux pendant 3 h. Après refroidissement on filtre, et les marcs sont à nouveau soumis au traitement précédent avec du méthanol neuf. On répète l'opération 3 fois. Les phases méthanoliques sont réunies et évaporées sous vide. Le résidu est agité avec 200 ml d'éther éthylique. L'insoluble dans l'éther est récupéré par filtration et dissous dans 3 l d'eau qui sont extraits par du butanol normal saturé d'eau (3 l puis 2 x 2 l).

Les phases butanoliques sont réunies et évaporées sous vide.

Le résidu (environ 150 g) est dissous dans 1,5 l d'eau et dialysé contre de l'eau pure pendant 4 jours avec renouvellement quotidien de l'eau. Le contenu de la cellule dialysée est lyophilisé.

Avantageusement, on réalise une purification supplémentaire : le lyophilisat est dissous dans 300 ml de méthanol et jeté dans 1,5 l d'éther. Le précipité constitué du mélange de saponines est récupéré par filtration et séché une nuit sous vide, en présence de P$_2$O$_5$. Le rendement moyen est de 30 g.

Exemple 3

Préparation de mélanges de saponines de racines de luzerne à pH contrôlé

On procède comme décrit à l'exemple 2.

Ensuite, de la résine échangeuse de cations Amberlite IRN 77 (50 g) est mise en suspension dans 100 ml d'eau et le mélange est placé dans une colonne de verre fermé par un robinet à sa partie inférieure.

La résine est régénérée par 90 ml d'acide chlorhydrique dilué au tiers jusau'à ce que l'éluant ait un pH acide.

La résine est alors rincée par 500 ml d'eau distillée jusqu'à pH neutre.

5,27 g du mélange de saponines obtenu à l'exemple 2 sont dissous dans un mélange de 30 ml d'eau et de 10 ml d'éthanol qui est ensuite passé sur la colonne de résine. L'éluat par l'eau de la colonne (200 ml) est séparé en deux. La moitié est congelée puis lyophilisée et donne 2,29 g de saponines sous forme acide que l'on recueille.

L'autre moitié est alcalinisée par une base, en particulier NH$_4$OH, jusqu'à pH 10. Cette partie est lyophilisée et donne 2,44 g de sel, en particulier d'ammonium, de saponines.

Exemple 4

Préparation d'un mélange de saponines de racines de Medicago romanica

On procède comme décrit à l'exemple 2 si ce n'est que l'on utilise des racines de Medicago romanica.

On obtient ainsi un extrait sec pulvérulent de saponines de racines de Medicago romanica.

Exemple 5

Préparation d'un mélange de saponines de tiges de Medicago arborea

On procède comme décrit à l'exemple 2 si ce n'est que l'on utilise des tiges finement broyées de Medicago arborea.

Exemple 6

Préparation d'un mélange de sapogénines de Medicago sativa

Le produit pulvérulent obtenu à la fin de l'exemple 2 consistant en un mélange de saponines de racines de Medicago sativa est dissous dans 250 ml de $HClO_4$ aqueux à 6,5%. La solution est répartie dans 3 tubes en verre qui sont scellés. Ces tubes sont portés à 140°C pendant 2 h dans une étuve. Après refroidissement, les tubes sont ouverts et le précipité formé recueilli par filtration sur verre fritté n° 3. Le précipité est lavé à l'eau distillée jusqu'à pH neutre et séché sous vide sur $P_2O_5$ dans un dessiccateur pendant 24 h.

Le précipité sec correspondant pèse 1,1 g environ et contient le mélange de sapogénines recherché.

Exemple 7

Préparation de liposomes contenant, dans leur bicouche, des sapogénines de Medicago sativa

On solubilise 0,01 g du mélange de sapogénines obtenues à l'exemple 6 dans 10 ml de méthanol et 60 ml ne dichlorométhane. On ajoute 5 g d'un mélange de lécithine de soja et de $\beta$-sitostérol dans un rapport en poids 9/1 et on agite jusqu'à solubilisation. On met dans un ballon d'évaporateur rotatif à 60°C pendant 1 h.

On reprend ensuite le film lipidique déposé sur la paroi interne du ballon avec 44,99 g d'eau distillée et on agite pendant 3 h à la température ambiante.

On réalise ensuite une sonication pendant 10 min à une puissance de 150 W à 4°C.

La taille moyenne des liposomes obtenus est de 125,2 ± 2,3 nm.

De préférence, on gélifie cette suspension de liposomes en la mélangeant à 50 g d'un gel de Carbopol 940® à 1,25% préparés séparément de façon classique. On obtient ainsi environ 100 g d'une suspension gélifiée de liposome encapsulant en partie un mélange de sapogénines de racines de Medicago sativa dont la concentration en sapogénines est d'environ 0,01% par rapport au poids total de la suspension gélifiée.

On peut observer qu'en fonction de dilution réalisée on peut obtenir des compositions de proportion variable en extrait de luzerne, ce qui constitue un mode de formulation particulièrement aisé.

Exemple 8

Préparation d'une suspension de liposomes gélifiée contenant un mélange de saponines de racines de luzerne

On prépare un mélange lipidique pulvérulent contenant 90% de lécithine de soja et 10% de bêta-sitostérol par le procédé décrit dans le document EP-B1-0 087 993 = US-A-4 508 703 mettant en oeuvre la technique d'atomisation.

On disperse sous agitation magnétique 4 g de ce mélange lipidique dans 96 g d'une solution aqueuse préparée par dissolution de 2 g de mélange de saponines selon l'exemple 2 dans 94 g d'eau. On maintient l'agitation pendant 2 h à température ambiante, puis on homogénéise aux ultrasons à 4°C pendant 10 min sous puissance de 150 W.

La taille moyenne des liposomes obtenus est de 73 nm environ.

De préférence, on gélifie cette suspension ce liposomes en la mélangeant à 100 g d'un gel de Carbopol 940® à 1,25% préparé séparément de façon classique. On obtient ainsi environ 200 g d'une suspension gélifiée de liposomes encapsulant en partie un mélange de saponines de racines de luzerne dont la concentration en saponines est de 1% environ par rapport au poids total de la suspension.

On peut observer qu'en fonction de dilution réalisée on peut obtenir des compositions de proportions variables en extraits de luzerne, ce qui constitue un mode de formulation particulièrement aisé.

Exemple 9

Essai d'activité de saponines de racines de Medicago sativa sur le renouvellement de l'épiderme

A. On réalise l'ensemencement de 50 000 kératinocytes humains transformés, dans des boîtes de pétri de diamètre 35 mm dans 2 ml de milieu EMEM-C (EMEM-C : milieu EMEM complété en acides aminés non-essentiels) auquel on a ajouté 1% en volume de SVF (SVF = sérum de veau foetal).

On procède à une incubation pendant 24 h à la température de 37°C.

Après ces 24 h d'incubation, on renouvelle les milieux par un milieu identique mais contenant respectivement différentes concentrations des produits à tester comme défini ci-après. Les essais sont réalisés en triple et des boîtes témoin sont prévues qui ne contiennent que le solvant du produit testé.

On réalise une incubation pendant 6 jours à la température de 37°C, et sous atmosphère d'air contenant 5% de $CO_2$.

Apres les 6 jours d'incubation, les cellules sont décollées à la trypsine et comptées avec l'appareil Coulter dénommé Coulter Compter.

Les produits testés ont les compositions suivantes :

Produit $I_1$ de l'invention

Il s'agit d'un mélange de saponines de racines de luzerne purifié, tel qu'obtenu à l'exemple 2, solubilisé dans du milieu EMEM-C.

Produit $I_2$ de l'invention

Il s'agit d'un mélange de saponines de feuilles de luzerne tel qu'obtenu selon un procédé identique à celui de l'exemple 2, solubilisé dans le DMSO.

Produit $I_3$

Il s'agit d'un extrait aqueux des parties aériennes de luzerne commercialement disponibles sous la dénomination "extrait aqueux de luzerne", chez Sochibo, également solubilisées dans le milieu de culture EMEM-C.

Ces produits sont introduits dans le milieu de culture à des concentrations non-cytotoxiques : 25 $\mu$g (extrait sec) par ml de milieu de culture, et 100 $\mu$g/ml de milieu de culture.

Les résultats obtenus sont répertoriés au tableau I, dans lequel sont indiqués : pour les boîtes témoin et les boîtes traitées par les produits $I_1$, $I_2$ et $I_3$, le nombre de moyen de cellules par boîte, après les six jours d'incubation, et pour les boîtes traitées, l'activité A des produits testés, calculée à partir du nombre de cellules dans chaque catégorie de boîte :

$$A = \frac{\text{bte traitée} - \text{bte témoin}}{\text{bte témoin}} \times 100$$

L'étude statistique a montré que les différences entre le nombre de cellules dans les boîtes traitées et les boîtes témoin, après incubation, étaient toutes significatives.

Tableau I

| Produit | Témoin Milieu Culture | Témoin DMSO | Racines luzerne (I₁) | | Feuilles luzerne (I₂) | | Extrait commercial (I₃) | |
|---|---|---|---|---|---|---|---|---|
| Concentration µg/ml | Nbre ℓ/bte | Nbre ℓ/bte | Nbre ℓ/bte* | Δ% | Nbre ℓ/bte* | Δ% | Nbre ℓ/bte* | Δ% |
| 0 | 145280 ± 6390 | 152813 ± 5794 | | | | | | |
| 25 | | | 173747 ± 1794 | +19,6 | 204333 ± 19656 | +34 | 171760 ± 2214 | +18 |
| 100 | | | 214153 ± 568 | +47 | 1755497 ± 9643 | +15 | 178580 ± 3304 | +23 |

* Nombre de cellules par boîte

On constate à partir du tableau I qu'à plus faible concentration, le produit I₂ (saponines de feuilles) selon l'invention est plus actif que l'extrait commercial I₃, tandis qu'à plus forte concentration, le produit I₁ - (saponines de racines) selon l'invention est le plus actif.

Il apparaît ainsi clairement que les saponines de luzerne selon l'invention présentent une meilleure efficacité sur la multiplication des cellules de l'épiderme qu'un extrait non spécifique des parties aériennes de luzerne.

B. On a également réalisé un essai d'activité sur la multiplication de kératinocytes humains avec deux autres produits, I₄ et I₅. Les conditions opératoires sont les mêmes que celles exposées ci-dessus, si ce n'est que l'on n'ajoute que 0,5% de SVF au milieu de culture EMEM-C.

Produit I₄ de l'invention

Il s'agit de la fraction sous forme acide du mélange de saponines de racines de luzerne préparée à l'exemple 3, solubilisée dans du DMSO.

Produit I₅ ce l'invention

Il s'agit de la fraction sous forme de sel d'ammonium du mélange de saponines de racines de luzerne préparée à l'exemple 3, solubilisée dans du milieu EMEM-C.

Les produits I₄ et I₅ sont testés à la concentration non-cytotoxique de 25 $\mu$g (extrait sec) par ml de milieu de culture.

Les résultats obtenus sont répertoriés au tableau II dans lequel sont indiqués le nombre moyen de cellules par boîte, après six jours d'incubation, et l'activité des produits I₄ et I₅ par rapport aux boîtes témoins.

Tableau II

| Témoin DMSO Nbre ℓ/bte* | Témoin EMEMC Nbre ℓ/bte* | I₄ fraction acide | | I₅ fraction sel | |
|---|---|---|---|---|---|
| | | Nbre ℓ/bte* | A% | Nbre ℓ/bte* | A% |
| 112 140 ± 1395 | 137 326 ± 4212 | 172 973 ± 10957 | 54% (S) | 160 140 ± 617 | 17% (S) |
| (S) = statistiquement significatif | | | | | |
| (*) = cellules par boîte | | | | | |

Les résultats du tableau II montrent clairement que les deux fractions sont toutes deux actives sur la multiplication des kératinocytes de l'épiderme humain. Toutefois la fraction du mélange de saponines de racines de luzerne sous forme acide semble beaucoup plus active que la fraction sous forme salifiée.

Exemple 10

Essai d'activité de saponines de racines de Medicago romanica sur le renouvellement de l' épiderme

On procède comme décrit à l'exemple 9 si ce n'est qu'on utilise comme produit de l'invention un mélange de saponines de racines de Medicago romanica tel qu'obtenu à l'exemple 4, solubilisé dans le DMSO.

Les résultats obtenus sont au tableau III ci-après :

Tableau III

| Produit testé | Nbre ℓ/bte* x $10^{-3}$ | Activité | Significabilité p < 0,05 |
|---|---|---|---|
| Témoin DMSO | 153 ± 4 | | |
| + 10 $\mu$g/ml | 164 ± 3 | + 7% | S |
| + 25 $\mu$g/ml | 172 ± 5 | + 12% | S |
| + 50 $\mu$g/ml de produit de l'invention (ex. 4) | 213 ± 9 | + 39% | S |

Il ressort clairement des résultats des essais rapportés au tableau III que le produit de l'invention, à savoir un mélange de saponines de racines de Medicago romanica présente une activité significative sur la multiplication des kératinocytes de l'épiderme humain.

Exemple 11

Essai d'activité de saponines de tiges de Medicago arborea sur le renouvellement de l'épiderme

On procède comme décrit à l'exemple 9, si ce n'est qu'on utilise un mélange de saponines de tiges de Medicago arborea obtenu à l'exemple 5 solubilisé dans le DMSO.

Les résultats obtenus sont répertoriés au tableau IV.

Tableau IV

| Produit testé | Nbre $\ell$/bte* x $10^3$ | Activité | Significabilité |
|---|---|---|---|
| Témoin DMSO | 153 ± 4 | | |
| + 10 $\mu$g/ml | 154 ± 5 | + 1% | NS |
| + 25 $\mu$g/ml | 227 ± 7 | + 48% | S |
| + 50 $\mu$g/ml de produit de l'invention (ex. 5) | 234 ± 5 | + 53% | S |

Il résulte clairement des résultats d'essai rapportés au tableau IV que les saponines de tiges de Medicago arborea sont significativement actives sur la multiplication des kératinocytes de l'épiderme humain.

Exemple 12

Mise en évidence de l'activité de saponines de racines de luzerne (Medicago sativa) sur la synthèse de collagène par des fibroblastes humains.

On effectue un prélèvement de peau d'une femme blanche de 55 ans au cours d'un lifting et on coupe la peau en fines bandelettes que l'on place dans une solution de trypsine à 0,25% pendant une nuit à 4°C.

Les bandelettes de derme ainsi obtenues sont découpées en fragments cubiques d'environ 1 mm de côté nommés explants. Ces explants sont déposés sur un film de milieu E 199 GIBCO, supplémenté avec 2 mM de L-glutamine et 10% de sérum de veau foetal à raison de 20 explants par boîte de culture de 100 mm de diamètre.

Les boîtes sont incubées à 37°C à l'atmosphère humide enrichie en $CO_2$ (5%). Le milieu est renouvelé deux fois par semaine.

Lorsque les cellules atteignent les confluences autour des explants, on change le milieu de culture, les fibroblastes sont extraits sans prélever les explants, par l'emploi d'un mélange trypsine 0,1%-EDTA 0,02% et les fibroblastes sont réimplantés dans un milieu de culture renouvelé. Certaines boîtes servent de témoins et reçoivent du milieu renouvelé sans le produit de l'invention, les autres boîtes sont divisées en deux lots recevant respectivement le produit de l'invention soit à 100 $\mu$g/ml soit à 250 $\mu$g/ml, ce produit de l'invention étant constitué par le mélange de saponines de racines de Medicago sativa obtenu à l'exemple 2.

On laisse incuber pendant 24 h en l'absence ou en présence du produit de l'invention à la concentration indiquée.

Après 24 h d'incubation, on procède à un dosage du collagène I sécrété par les fibroblastes dans le surnageant par une méthode similaire à celle décrite par RENNARD S.I., et al. dans Anal. Biochem. (1980), 104, 205-214.

EP 0 558 509 B1

Les résultats obtenus par cette méthode de desage sont répertoriés au tableau V ci-après.

## Tableau V

| Résultats | Collagène I secrété par fibroblastes (ng/10000 cellules/24 h | % de stimu- lation | Signifi- cativité p < 0,05 |
|---|---|---|---|
| Culture témoin sans produit | 269 ± 28,9 | 0 | |
| Produit de l'invention (ex. 2) 100 µg/ml 250 µg/ml | 306,9 ± 34,6 317,2 ± 25,8 | 14,1% 17,9% | S S |

Au tableau V, on a exprimé les quantités de collagène I secrétées par les fibroblastes en ng/10000 cellules/24 h.

On observera à partir du tableau V que le mélange de saponines de racines de Medicago sativa produit une activité significative de stimulation de la synthèse de collagène I par les fibroblastes.

A ce sujet, Shuster et al., dans Br. J. Dermatol. (1975), 93, 639-643, ayant pour titre "The Influence of age, and sex on skin thickness, skin collagen and density", ont mis en évidence une diminution du contenu on collagène de la peau entre 15 et 93 ans. De plus, le collagène est à la base de l'architecture du derme d'où l'interêt de produit capable d'exercer un effet de stimulation de la synthèse de collagène.

Exemple 13

Mise en évidence de l'activité des saponines de la luzerne selon l'invention sur le système pilaire

Le test repose sur l'étude de l'activité des produits selon l'invention sur le cycle pilaire de rats Sprague Dawley tous âgés de 23 jours. A cet âge, les cycles pilaires de tous les animaux sont encore synchrones. Les rats sont rasés, au niveau de la partie inférieure du dos, le $24^{ème}$ jour.

On applique ensuite (à partir du $25^{ème}$ jour) jusqu'au $65^{ème}$ jour, 6 jours sur 7, les produits à tester, à une dose évoluant avec le poids des animaux. Cette dose est de 0,5 ml au 25e jour, et atteint 2 ml au 65e jour.

A intervalle de temps sensiblement régulier (environ tous les 3 jours) on prélève à l'aide d'une pince à épiler une touffe de poils sur le côté gauche de l'animal au niveau du flanc. On observe 10 poils pris au hasard dans cette touffe, et on compte le nombre de poils en phase anagène. On détermine ainsi par lot de 10 animaux le pourcentage de poils en phase anagène (phase de croissance), en fonction du temps.

L'étude est effectuée sur 30 rats répartis en 3 lots de 10 animaux. Le premier lot reçoit une préparation contenant 0,1% du produit de l'exemple 2 (mélange de saponines de racines de luzerne) dans un excipient. Composition de l'excipient pour 100 g : eau 15 g, propylène glycol 20 g, alcool 95% 65 g. Le second ne reçoit que l'excipient précité . Le troisième lot est le lot témoin ne recevant aucun produit.

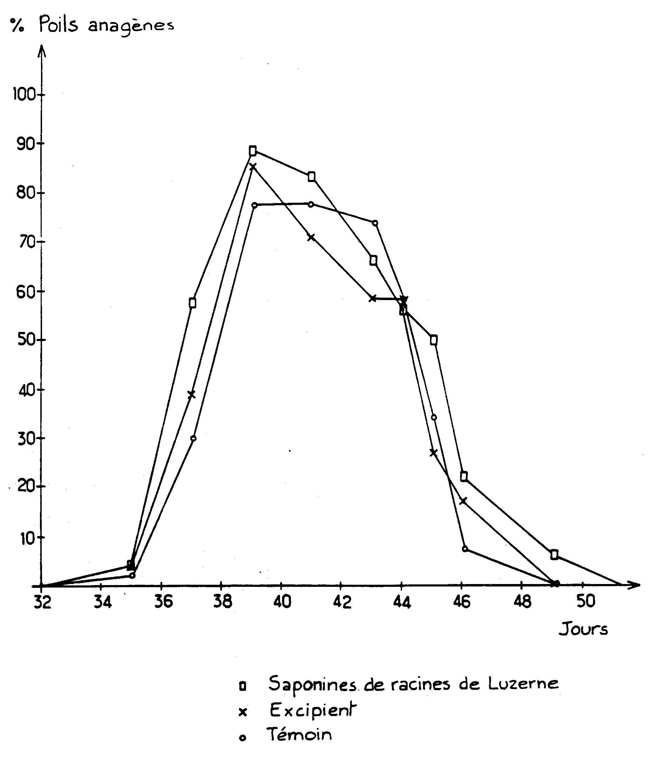

Les résultats de cette étude trichocinétique (trichogramme en fonction du temps) sont reportés, en valeurs moyennes, au tableau VI et représentés sur la figure unique annexée.

13

Tableau VI

| | Pourcentage poils anagènes | | | | | | | |
|---|---|---|---|---|---|---|---|---|
| | 37 jours | 39 j. | 41 j. | 43 j. | 44 j. | 45 j. | 46 j. | 49 j. |
| Composition saponines + excipient (invention) | 59 | 89 | 83 | 66 | 56 | 50 | 22 | 6 |
| Excipient seul | 39 | 86 | 71 | 58 | 59 | 27 | 17 | 0 |
| Témoin | 30 | 78 | 78 | 74 | 57 | 31 | 7 | 0 |

Sur cette figure, on a représenté en ordonnée le pourcentage de poils en phase anagène, et en abcisse le nombre de jours.

La courbe joignant les carrés correspond aux résultats obtenus avec le mélange de saponines de racine de luzerne de l'exemple 2 selon l'invention, la courbe joignant les croix est obtenue avec l'excipient et enfin, la courbe joignant les points est obtenue avec le lot témoin ne recevant aucun produit.

On observe que le nombre de poils en phase anagène croît beaucoup plus rapidement dans le lot recevant le mélange de saponines de racine de luzerne selon l'invention par rapport au lot recevant l'excipient ou encore par rapport au lot témoin. On observe également que la phase anagène se prolonge plus longtemps.

Ainsi, il est clair que les extraits de luzerne selon l'invention, par rallongement de la durée de la phase anagène, retardent très nettement la chute des cheveux et favorisent la repousse.

On donnera ci-après divers exemples de formulation de compositions cosmétiques ou pharmaceutiques, favorisant la repousse des cheveux et/ou retardant leur chute et/ou favorisant le renouvellement ou la croissance de l'épiderme.

Exemple 14

Gel régulateur de l'épiderme

On dissout 0,2 g de saponines de racines de Medicago romanica tel qu'obtenu à l'exemple 4 dans 49,2 g d'eau distillée, puis on gélifie cette solution en ajoutant 50 g d'un gel neutralisé de Carbopol 940® à 3%.

On peut appliquer ce gel 2 fois par jour par cure de 4 mois.

Exemple 15

Gel contour de l'oeil

On prépare la composition suivante :

| Composant A | |
|---|---|
| - saponine de Medicago sativa telle qu'obtenue à l'exemple 2 | 0,20 |
| - vitamine E succinate polyoxyéthyléné | 0,10 |
| - α-Bisabolol | 0,1 |
| - acide hyaluronique | 0,20 |
| - propylèneglycol | 4,00 |
| - eau | 83,4 |

| Composant B | |
|---|---|
| - gel de Carbopol 980 à 2,5% | 12,00 |

On réalise tout d'abord le mélange dans l'eau de tous les composants A et on introduit le composant B en obtenant ainsi un gel qui peut être utilisé pour son activité anti-relâchement du contour de l'oeil deux fois par jour.

Exemple 16

Mascara traitant favorisant la pousse des cils

On prépare la composition suivante :

| | |
|---|---|
| - mélange de saponines de Médicago sativa tel qu'obtenu à l'exemple 2 | 0,2 |
| - monostéarate de glycérol | 10 |
| - acide stéarique | 10 |
| - triéthanolamine | 5 |
| - myristate d'isopropyle | 15 |
| - cire d'abeilles | 25 |
| - eau distillée | 27,8 |
| - gélatine | 2 |
| - oxyde de fer noir | 5 |

Cette composition formant mascara est utilisée de manière classique sur les cils et procure l'avantage de favoriser la pousse des cils.

Exemple 17

Gel liposomal anti-vieillissement

On utilise le gel tel que préparé selon l'exemple 7 mais en remplaçant l'eau distillée par une solution aqueuse contenant 0,2% en poids d'hydroxyproline.
Ce gel est appliqué sur la peau et procure un raffermissement de celle-ci et une atténuation des rides.

Exemple 18

Lotion anti-chute des follicules pileux, en particulier des cheveux

On prépare la composition suivante :

| | |
|---|---|
| - mélange de saponines de racines de luzerne sous forme acide (exemple 3) | 0,1 g |
| - panthénol | 0,1 g |
| - hydrolysat de kératine | 0,3 g |
| - parfum | 0,1 g |
| - alcool à 20% qsp | 100 g |

Exemple 19

Gel coiffant anti-chute

On prépare la composition suivante :

| | |
|---|---|
| - extrait de parties aériennes de luzerne selon l'exemple 1) | 0,3 g |
| - gel de Carbopol 940® 1,50% neutralisé | 45 g |
| - Phytantriol® | 0,1 g |
| - complexe protéine-zinc | 0,1 g |
| - conservateur | 0,05 g |
| - eau qsp | 100 g |

On dissout d'abord le mélange de saponines dans l'eau puis on ajoute le Phytantriol, le complexe de protéine-zinc, l'agent conservateur et enfin le Carbopol 940®.

Le gel obtenu est appliqué mation et soir sur les zones de chute des follicules pileux pendant 6 mois.

Exemple 20

Gel traitant contre l'alopécie séborrhéique

On prépare la composition suivante :

| | |
|---|---|
| - mélange de saponines des racines de luzerne de l'exemple 2 | 0,2 g |
| - parfum | 0,1 g |
| - complexe protéine-zinc | 0,05 g |
| - gel Carbopol 940® 1,50% | 45 g |
| - eau qsp | 100 g |

On procède comme décrit à l'exemple 9 pour préparer le gel.
On peut appliquer ce gel matin et soir sur les cheveux pendant 6 mois.

Exemple 21

Crème traitante contre la sénescence cutanée

On prépare une crème à 0,2% d'extrait de racines de luzerne de la manière suivante :

| Composition A (pourcentage en poids) | |
|---|---|
| - Tefosse 1500® de chez Gattefossé | 7,00 |
| - alcool cétylique | 2,00 |
| - Primol 352® de chez ESSO | 25,00 |

| Composition B (pourcentage en poids) | |
|---|---|
| - eau déminéralisée | 54,40 |
| - Carbopol 940® de chez Polyplastic | 0,30 |
| - triéthanolamine | 0,30 |

| Composition C (pourcentage en poids) | |
|---|---|
| - Germaben II® de chez Seppic | 0,80 |

| Composition D (pourcentage en poids) | |
|---|---|
| - eau déminéralisée | 10,00 |
| - mélange de saponines de racines de luzerne de l'exemple 2 | 0,20 |

On chauffe la composition A à 75°C puis on disperse le Carbopol 940® dans l'eau de la composition B. On neutralise le gel avec de la triéthanolamine. On chauffe la solution B à 75°C. On réalise une émulsion de la composition de A dans la composition B à 75°C. On refroidit sous agitation jusqu'à 40°C.
On ajoute ensuite la composition C. On réalise le prémélange des composants de la composition D puis on ajoute la composition D à la formule. On homogénéise et on refroidit jusqu'à la température ambiante en obtenant ainsi une crème.
On peut appliquer cette crème deux fois par jour par cure de 6 mois pour obtenir.

**Revendications**

1.  Utilisation d'au moins une saponine triterpénique de Medicago, ou d'au moins une sapogénine correspondante ou d'un extrait végétal en contenant, pour la fabrication d'une composition cosmétique ou pharmaceutique, notamment dermatologique, destinée en particulier à favoriser le renouvellement de l'épiderme, à stimuler la repousse des cheveux ou à retarder leur chute et à lutter contre les effets du vieillissement sur l'état de la peau et de la chevelure.

2.  Utilisation selon la revendication 1, caractérisée en ce que la saponine et l'extrait végétal précités sont obtenus par extraction à partir de parties aériennes, telles que feuilles ou tiges, ou de racines de Medicago, de préférence à partir de racines de cette plante.

3.  Utilisation selon la revendication 1, caractérisée en ce que la sapogénine et l'extrait végétal précités sont obtenus à partir de cals obtenus par culture in vitro de tissus de Medicago, en particulier à partir de tissus de racines de cette plante.

4.  Utilisation selon l'une quelconque des revendications 1 à 3, caractérisée en ce que la saponine précitée est choisie parmi celles qui comportent un groupement carboxylique sous forme acide.

5.  Utilisation selon l'une des revendications 1 à 4, caractérisée en ce que la plante Medicago précitée est choisie parmi le groupe constitué par : Medicago sativa, Medicago lupulina, Medicago truncatula, Medicago laciniata, Medicago littoralis, Medicago falcata, Medicago media, Medicago minima, Medicago varia, Medicago arborea et Medicago romanica.

6.  Utilisation selon l'une des revendications 1 à 5, caractérisée en ce que l'extrait végétal précité est obtenu à partir de matières sèches, constituées de préférence de racines de Medicago, au moyen d'un solvant choisi parmi le groupe constitué par : l'eau, les alcools comportant de préférence de 1 à 4 atomes de carbone, les esters organiques comportant de préférence de 3 à 6 atomes de carbone ; ou d'un solvant mixte à base d'un mélange quelconque des solvants précités.

7.  Utilisation selon la revendication 6, caractérisée en ce que le solvant d'extraction est choisi parmi le groupe consistant du méthanol, de l'éthanol, d'un mélange méthanol-eau ou un mélange éthanol-eau.

8.  Utilisation d'un mélange de saponines selon l'une des revendications 1 à 5, caractérisée en ce que ledit mélange est en particulier obtenu par précipitation de l'extrait végétal selon la revendication 6 ou 7, dans un solvant apolaire de préférence miscible avec le solvant d'extraction précité.

9.  Utilisation selon la revendication 8, caractérisée en ce que le mélange de saponines précité subit un traitement supplémentaire destiné à faire passer sous forme acide les saponines qui comportent un groupement carboxylique salifié.

10. Utilisation selon l'une des revendications 1 à 9, caractérisée en ce que les sapogénines sont obtenues à partir des saponines par hydrolyse des liaisons glycosidiques des saponines.

11. Utilisation selon la revendication 10, caractérisée en ce que l'hydrolyse précitée est une hydrolyse acide, notamment avec des acides halogénés tels que l'acide perchlorique, l'acide fluoborique, l'acide trifluoroacétique.

12. Utilisation selon la revendication 10 ou 11, caractérisée en ce que la sapogénine précitée est choisie parmi le groupe constitué par l'acide lucernique, l'acide médicagénique, l'acide zanhique, la bayogénine, l'hédéragénine, les sojasapogénols A, B, C et E.

13. Utilisation selon l'une des revendications 1 à 12, caractérisée en ce que la saponine précitée, la sapogénine correspondante précitée ou l'extrait de luzerne précité, est au moins en partie incorporé dans une phase lamellaire lipidique hydratée ou dans des vésicules de type liposomes.

14. Utilisation selon la revendication 13, caractérisée en ce que la saponine précitée est introduite dans la phase aqueuse de la phase lamellaire lipidique hydratée ou des liposomes à une concentration

EP 0 558 509 B1

comprise entre 0,01% et 5% en poids du poids total de ladite phase aqueuse.

15. Utilisation selon la revendication 9, caractérisée en ce que la sapogénine précitée est incorporée dans la phase lipidique de la phase lamellaire hydratée ou des liposomes à une concentration comprise entre 0,01% et 30% en poids de ladite phase lipidique, et de préférence entre 0,01% et 10% en poids de cette phase lipidique.

16. Composition cosmétique ou pharmaceutique, notamment dermatologique, destinée en particulier à favoriser le renouvellement de l'épiderme, à stimuler la repousse des cheveux ou à retarder leur chute, et à lutter contre les effets du vieillissement sur l'état de la peau et de la chevelure, caractérisée en ce qu'elle comprend, à titre d'ingrédient actif, une quantité efficace d'au moins une saponine triterpénique de Medicago ou d'au moins une sapogénine correspondante ou d'au moins un extrait végétal en contenant, éventuellement dans un excipient, support ou véhicule cosmétiquement ou pharmaceutiquement acceptable.

17. Composition selon la revendication 16, caractérisée en ce que la saponine précitée, ou la sapogénine précitée, ou l'extrait précité est obtenu par extraction à partir de parties aériennes telles que feuilles ou tiges, ou de racines de Medicago, de préférence à partir de racines de cette plante.

18. Composition selon la revendication 16 ou 17, caractérisée en ce que la saponine ou la sapogénine précitée ou l'extrait végétal précité peut être au moins en partie incorporé dans des phases lamellaires lipidiques hydratées ou dans des vésicules de type liposomes.

19. Composition selon l'une des revendications 16 à 18, caractérisée en ce que la concentration en saponine ou en sapogénine précitée ou en extrait végétal précité est comprise entre 0,001% et 5%, encore mieux entre 0,01 et 2% en poids, par rapport au poids total de la composition cosmétique ou pharmaceutique.

20. Composition selon l'une des revendications 16 à 19, caractérisée en ce qu'elle comprend en outre au moins une autre substance active en une concentration efficace, choisie parmi les xanthines, Les vitamines, en particulier les vitamines A, B ou E la tyrosine ou ses dérivés comme par exemple tyrosinate de glucose, la malyl tyrosine, la quinine ou ses dérivés, les rubéfiants tels que le nicotinate de méthyle, un surnageant de culture de fibroblaste de papille, des hydrolysats de kératine, des oligo-éléments tels que le zinc, sélénium, cuivre, des inhibiteurs de 5-$\alpha$-réducteurs tels que de progestérone, acétate de cyprotérone, Minoxidil, acide azélaïque et ses dérivés, 1,4-méthyl-4-azastéroïde, en particulier la 17-$\beta$-N,N-diéthylcarbamoyle-4-méthyl-4-aza-5-$\alpha$-androstan-3-one, ou encore un extrait de Serenoa Repens ; cette substance étant éventuellement incorporée au moins en partie dans des phases lamellaires lipidiques hydratées ou dans des vésicules type liposomes.

21. Composition selon l'une des revendications 16 à 20, caractérisée en ce que la saponine et l'extrait végétal précités sont obtenus à partir de cals obtenus par culture in vitro de tissu de Medicago, en particulier à partir de tissu de racines de cette plante.

22. Composition selon l'une des revendications 16 à 21, caractérisée en ce que la saponine précitée est choisie parmi celles qui comportent un groupement carboxylique sous forme acide.

23. Composition selon l'une des revendications 16 à 22, caractérisée en ce qu'elle comprend un mélange de saponines en particulier obtenu par précipitation de l'extrait végétal dans un solvant apolaire de préférence miscible avec le solvant d'extraction utilisé.

24. Procédé de traitement cosmétique destiné notamment à favoriser le renouvellement de l'épiderme, à stimuler la repousse des cheveux ou à retarder leur chute, et à lutter contre les effets du vieillissement sur l'état de La peau et de la chevelure, caractérisé en ce qu'il comprend l'application topique, à une quantité efficace, pour réaliser ledit effet recherché, d'au moins une saponine de Medicago ou d'au moins une sapogénine correspondante ou d'un extrait végétal en contenant.

25. Procédé selon la revendication 24, caractérisé en ce qu'il comprend en premier lieu l'incorporation d'au moins une saponine précitée ou d'au moins une sapogénine correspondante ou d'un extrait végétal en

18

EP 0 558 509 B1

contenant, au moins en partie dans des phases lamellaires lipidiques hydratées ou dans des vésicules type liposomes, puis leur mélange dans un excipient, véhicule ou support cosmétiquement acceptable, avant de procéder à ladite application topique.

## Claims

1. Use of at least one Medicago triterpene saponin or at least one corresponding sapogenin, or of one plant extract in which it is present for preparing a cosmetic or pharmaceutical composition, notably a dermatological composition, intended in particular for promoting renewal of the epidermis, stimulating hair regrowth or delaying hair loss, and for combating the effects of ageing on the state of the skin and scalp.

2. Use according to claim 1, characterized in that said saponin and plant extract are obtained by extraction from aerial parts, such as leaves or stems, or roots of Medicago, preferably from roots of this plant.

3. Use according to claim 1, characterized in that said sapogenin and plant extract are obtained from calluses obtained by in vitro culture of tissue of Medicago, in particular from root tissues of this plant.

4. Use according to any one of claims 1 to 3, characterized in that saponin is selected from those containing a carboxyl group in acid form.

5. Use according to one of claims 1 to 4, characterized in that said Medicago plant is selected from the group constituted by: Medicago sativa, Medicago lupulina, Medicago truncatula, Medicago laciniata, Medicago littoralis, Medicago falcata, Medicago media, Medicago minima, Medicago varia, Medicago arborea and Medicago romanica.

6. Use according to one of claims 1 to 5, characterized in that said plant extract is obtained from dry materials, preferably constituted of Medicago roots, using a solvent selected from the group constituted by : water, alcohols preferably those containing 1 to 4 carbon atoms, the organic esters preferably comprising 3 to 6 carbon atoms; or of a mixed solvent based on any mixture of said solvents.

7. Use according to claim 6, characterized in that the extraction solvent is selected from the group consisting of methanol, ethanol, a methanol-water mixture or an ethanol-water mixture.

8. Use of a mixture of saponins according to one of claims 1 to 5, characterized in that said mixture is obtained in particular by precipitation of the plant extract according to claim 6 or 7, in an apolar solvent preferably miscible with said extraction solvent.

9. Use according to claim 8, characterized in that said saponin mixture undergoes an additional treatment intended to convert the saponins which contain a salified carboxylic group into acid form.

10. Use according to one of claims 1 to 9, characterized in that the sapogenins are obtained from saponins by hydrolysis of the glycosidic bonds of the saponins.

11. Use according to claim 10, characterized in that said hydrolysis is an acid hydrolysis, notably with halogen acids such as perchloric acid, fluoboric acid, trifluoroacetic acid.

12. Use according to claim 10 or 11, characterized in that said sapogenin is selected from the group constituted by lucernic acid, medicagenic acid, zanhic acid, bayogenin, hederagenin, soyasopogenols A, B, C and E.

13. Use according to one of claims 1 to 12, characterized in that said saponin, said corresponding sapogenin or said lucern extract, is at least partly incorporated in a hydrated lipidic lamellar phase or in liposome-type vesicles.

14. Use according to claim 13, characterized in that said saponin is introduced into the aqueous phase of the hydrated lipidic phase or of the liposomes at a concentration of between 0.01 % and 5 % by weight

19

of the total weight of said aqueous phase.

15. Use according to claim 9, characterized in that said sapogenin is incorporated into the lipidic phase of the hydrated lamellar phase or of the liposomes at a concentration of between 0.01 % and 30 % by weight of said lipidic lamellar phase, and preferably between 0.01 % and 10 % by weight of said lipidic phase.

16. Cosmetic or pharmaceutical composition, notably dermatological, intended in particular for promoting renewal of the epidermis, stimulating hair regrowth or delaying hair loss, and for combating the effects of ageing on the state of the skin and scalp, characterized in that it comprises, as active ingredient, an effective quantity of at least one Medicago triterpene saponin or at least one corresponding sapogenin, or at least one plant extract in which it is present, if appropriate in a cosmetically or pharmaceutically acceptable excipient, carrier or vehicle.

17. Use according to claim 16, characterized in that said saponin or said sapogenin, or said extract is obtained by extraction from aerial parts, such as leaves or stems, or roots of Medicago, preferably from roots of this plant.

18. Composition according to claim 16 or 17, characterized in that said saponin or sapogenin or said plant extract can be at least partially incorporated into hydrated lipidic lamellar phases or into vesicles of the liposome type.

19. Composition according to one of claims 16 to 18, characterized in that said concentration of saponin or sapogenin or of said plant extract is comprised between 0.001 % and 5 %, and better still between 0.01 and 2 % by weight, with respect to the total weight of the cosmetic or pharmaceutical composition.

20. Composition according to one of claims 16 to 19, characterized in that it further comprises an effective concentration of at least one other active substance selected from xanthines, vitamins, in particular vitamins A, B or E, tyrosine or its derivatives, for example glucose tyrosinate, malyltyrosine, quinine or its derivatives, rubefacients such as methyl nicotinate, a papilla fibroblast culture supernatant, keratin hydrolyzates, trace elements such as zinc, selenium, copper, 5-alpha-reducer inhibitors such as progesterone, cyproterone acetate, Minoxidil, azelaic acid and its derivatives, a 1,4-methyl-4-azasteroid, in particular 17-beta-N,N-diethylcarbamoyl-4-methyl-4-aza-5-alpha-androstan-3-one, or else an extract of Serenoa Repens; this substance optionally being at least partially incorporated into hydrated lipidic lamellar phases or into vesicles of the liposome type.

21. Composition according to one of claims 16 to 20, characterized in that said saponin and plant extract are obtained from calluses obtained by the in vitro culture of tissue of Medicago, in particular from root tissues of this plant.

22. Composition according to one of claims 16 to 21, characterized in that said saponin is selected from those containing a carboxyl group in the acid form.

23. Composition according to one of claims 16 to 22, characterized in that it comprises a mixture of saponins obtained in particular by precipitation of the plant extract in an apolar solvent which is preferably miscible with the extraction solvent used.

24. Method for the cosmetic treatment notably for promoting renewal of the epidermis, for hair treatment for promoting hair regrowth or delaying hair loss, and for combating the effects of ageing on the state of the skin and scalp, which method comprises applying topically, an efficient amount for performing said effect, of at least one Medicago saponin or at least one corresponding sapogenin, or a plant extract containing it.

25. Method according to claim 24, characterized in that it comprises firstly at least partially incorporating at least one said saponin or at least one corresponding sapogenin, or a plant extract in which it is present, at least partly into hydrated lipidic lamellar phases or into vesicles of the liposome type and then mixing them with a cosmetically acceptable excipient, vehicle or carrier, prior to performing said application.

EP 0 558 509 B1

**Patentansprüche**

1. Verwendung eines oder mehrerer Triterpenoid-Saponine aus Medicago oder eines oder mehrerer entsprechender Sapogenine oder eines oder mehrerer diese Verbindungen enthaltender Pflanzenextrakte zur Herstellung kosmetischer oder pharmazeutischer und insbesondere dermatologischer Zusammensetzungen, die insbesondere zur Begünstigung der Erneuerung der Epidermis, zur Stimulation des Nachwachsens neuer Haare oder zur Verzögerung des Haarausfalls und zur Bekämpfung von Alterungseffekten auf den Zustand der Haut und des Haarwuchses vorgesehen sind.

2. Verwendung nach Anspruch 1, dadurch gekennzeichnet, daß das Saponin und der Pflanzenextrakt durch Extraktion aus oberirdischen Teilen, wie Blättern oder Stengeln, oder aus Wurzeln von Medicago erhalten sind, vorzugsweise aus den Wurzeln dieser Pflanze.

3. Verwendung nach Anspruch 1, dadurch gekennzeichnet, daß das Sapogenin und der Pflanzenextrakt aus durch Invitro-Kultivierung von Gewebe von Medicago, insbesondere aus Wurzelgewebe dieser Pflanze, erhaltenem Kallus hergestellt sind.

4. Verwendung nach einem der Ansprüche 1 bis 3, dadurch gekennzeichnet, daß das Saponin unter den Saponinen ausgewählt ist, die eine Carboxylgruppe in der Säureform enthalten.

5. Verwendung nach einem der Ansprüche 1 bis 4, dadurch gekennzeichnet, daß die Medicago-Pflanze unter Medicago sativa, Medicago lupulina, Medicago truncatula, Medicago laciniata, Medicago littoralis, Medicago falcata, Medicago media, Medicago minima, Medicago varia, Medicago arborea und Medicago romanica ausgewählt ist.

6. Verwendung nach einem der Ansprüche 1 bis 5, dadurch gekennzeichnet, daß der Pflanzenextrakt aus Trockenmaterial, das vorzugsweise aus Wurzeln von Medicago besteht, mit Hilfe eines Lösungsmittels, das unter Wasser, Alkoholen mit vorzugsweise 1 bis 4 C-Atomen und organischen Estern mit vorzugsweise 3 bis 6 C-Atomen ausgewählt ist, oder eines Mischlösungsmittels auf der Basis eines beliebigen Gemisches der genannten Lösungsmittel erhalten ist.

7. Verwendung nach Anspruch 6, dadurch gekennzeichnet, daß das Extraktionslösungsmittel unter Methanol, Ethanol, Methanol-Wasser-Gemischen und Ethanol-Wasser-Gemischen ausgewählt ist.

8. Verwendung eines Gemischs von Saponinen nach einem der Ansprüche 1 bis 5, dadurch gekennzeichnet, daß das Gemisch insbesondere durch Fällung des Pflanzenextrakts nach Anspruch 6 oder 7 in einem apolaren Lösungsmittel, das vorzugsweise mit dem genannten Extraktionslösungsmittel mischbar ist, erhalten ist.

9. Verwendung nach Anspruch 8, dadurch gekennzeichnet, daß das Gemisch von Saponinen einer zusätzlichen Behandlung unterzogen ist, die dazu dient, die Saponine, die eine in Salzform vorliegende Carboxylgruppe aufweisen, in die entsprechende Säureform überzuführen.

10. Verwendung nach einem der Ansprüche 1 bis 9, dadurch gekennzeichnet, daß die Sapogenine aus den Saponinen durch Hydrolyse ihrer glycosidischen Bindungen erhalten sind.

11. Verwendung nach Anspruch 10, dadurch gekennzeichnet. daß die Hydrolyse eine saure Hydrolyse ist, insbesondere mit halogenhaltigen Säuren, wie Perchlorsäure, Fluorborsäure und Trifluoressigsäure.

12. Verwendung nach Anspruch 10 oder 11, dadurch gekennzeichnet, daß das Sapogenin unter Luzernesäure, Medicagensäure, Zanhsäure, Bayogenin, Hederagenin und den Soja-Sapogenolen A, B, C und E ausgewählt ist.

13. Verwendung nach einem der Ansprüche 1 bis 12, dadurch gekennzeichnet, daß das Saponin, das entsprechende Sapogenin oder der Luzerneextrakt mindestens zum Teil in eine wasserhaltige lamellare Lipidphase oder in Vesikel vom Liposomtyp eingebracht werden.

21

**14.** Verwendung nach Anspruch 13, dadurch gekennzeichnet, daß das Saponin in die wäßrige Phase der wasserhaltigen lamellaren Lipidphase oder der Liposomen in einer Konzentration von 0,01 bis 5 Gew.-%, bezogen auf das Gesamtgewicht der wäßrigen Phase, eingebracht wird.

**15.** Verwendung nach Anspruch 9, dadurch gekennzeichnet, daß das Sapogenin in die Lipidphase der wasserhaltigen lamellaren Phase oder der Liposomen in einer Konzentration von 0,01 bis 30 Gew.-% und vorzugsweise 0,01 bis 10 Gew.-%, bezogen auf das Gewicht der Lipidphase, eingebracht wird.

**16.** Kosmetische oder pharmazeutische Zusammensetzungen, die insbesondere zur Begünstigung der Erneuerung der Epidermis, zur Stimulation des Nachwachsens neuer Haare oder zur Verzögerung des Haarausfalls und zur Bekämpfung von Alterungseffekten auf den Zustand der Haut und des Haarwuchses vorgesehen sind, dadurch gekennzeichnet, daß sie als Wirkstoff eine wirksame Menge eines oder mehrerer Triterpenoid-Saponine aus Medicago oder eines oder mehrerer entsprechender Sapogenine oder eines oder mehrerer diese Verbindungen enthaltender Pflanzenextrakte, ggfs. in einem kosmetisch oder pharmazeutisch geeigneten Excipiens, Träger oder Vehikel, enthalten.

**17.** Zusammensetzungen nach Anspruch 16, dadurch gekennzeichnet, daß das Saponin, das Sapogenin oder der Pflanzenextrakt durch Extraktion aus oberirdischen Teilen, wie Blättern oder Stengeln, oder aus Wurzeln von Medicago erhalten sind, vorzugsweise aus Wurzeln dieser Pflanze.

**18.** Zusammensetzungen nach Anspruch 16 oder 17, dadurch gekennzeichnet, daß das Saponin, das Sapogenin oder der Pflanzenextrakt mindestens zum Teil in wasserhaltige lamellare Lipidphasen oder Vesikel vom Liposomtyp eingebracht ist.

**19.** Zusammensetzungen nach einem der Ansprüche 16 bis 18, dadurch gekennzeichnet, daß die Konzentration an Saponin oder Sapogenin oder an dem Pflanzenextrakt im Bereich von 0,001 bis 5 Gew.-% und noch günstiger im Bereich von 0,01 bis 2 Gew.-% liegt, bezogen auf das Gesamtgewicht der kosmetischen oder pharmazeutischen Zusammensetzung.

**20.** Zusammensetzungen nach einem der Ansprüche 16 bis 19, dadurch gekennzeichnet, daß sie ferner mindestens einen weiteren Wirkstoff in einer wirksamen Konzentration enthalten, der unter den Xanthinen, den Vitaminen, insbesondere den Vitaminen A, B und E, Tyrosin und seinen Derivaten, wie beispielsweise Glucosetyrosinat und Malyltyrosin, Chinin und seinen Derivaten, Rubefacientien, wie Methylnicotinat, einem Überstand einer Kultur von Papillen-Fibroplasten, Keratinhydrolysaten, Spurenelementen, wie Zink, Seien und Kupfer, Inhibitoren von 5-$\alpha$-Reduktionsmitteln, wie Progesteron, Cyproteronacetat, Minoxidil, Azelainsäure und ihren Derivaten, 1,4-Methyl-4-azasteroiden, insbesondere 17-$\beta$-N,N-Diethylcarbamoyl-4-methyl-4-aza-5- - androstan-3-on, sowie Extrakten von Serenoa Repens ausgewählt ist, wobei diese Substanz ggfs. mindestens zum Teil in wasserhaltige lamellare Lipidphasen oder Vesikel vom Liposomtyp eingebracht ist.

**21.** Zusammensetzungen nach einem der Ansprüche 16 bis 20, dadurch gekennzeichnet, daß das Saponin und der Pflanzenextrakt aus durch In-vitro-Kultivierung von Gewebe von Medicago, insbesondere aus Wurzelgewebe dieser Pflanze, erhaltenem Kallus hergestellt sind.

**22.** Zusammensetzung nach einem der Ansprüche 16 bis 21, dadurch gekennzeichnet, daß das Saponin unter den eine Carboxylgruppe in der Säureform enthaltenden Saponinen ausgewählt ist.

**23.** Zusammensetzungen nach einem der Ansprüche 16 bis 22, dadurch gekennzeichnet, daß sie ein Gemisch von Saponinen enthalten, das insbesondere durch Fällung des Pflanzenextrakts in einem apolaren Lösungsmittel, das vorzugsweise mit dem verwendeten Extraktionslösungsmittel mischbar ist, erhalten ist.

**24.** Verfahren zur kosmetischen Behandlung, das insbesondere zur Begünstigung der Erneuerung der Epidermis, zur Stimulation des Nachwachsens neuer Haare oder zur Verzögerung des Haarausfalls und zur Bekämpfung von Alterungseffekten auf den Zustand der Haut und des Haarwuchses vorgesehen ist, gekennzeichnet durch die topische Verabreichung eines oder mehrerer Saponine aus Medicago oder eines oder mehrerer entsprechender Sapogenine oder eines diese Substanzen enthaltenden Pflanzenextrakts in einer wirksamen Menge, um die angestrebte Wirkung zu erreichen.

**25.** Verfahren nach Anspruch 24, dadurch gekennzeichnet, daß zunächst ein oder mehrere Saponine oder ein oder mehrere entsprechende Sapogenine oder ein Pflanzenextrakt, der diese Substanzen enthält, zumindest zum Teil in wasserhaltige lamellare Lipidphasen oder Vesikel vom Liposomtyp eingebracht werden, worauf diese in ein kosmetisch geeignetes Excipiens oder Vehikel oder einen entsprechenden Träger eingemischt werden, bevor die topische Verabreichung durchgeführt wird.

% Poils anagènes

□  Saponines de racines de Luzerne
x  Excipient
○  Témoin